# EUROPEAN PATENT SPECIFICATION

(11) **EP 1 957 102 B1**
(45) Date of publication and mention of the grant of the patent: **18.01.2012**
(21) Application number: 06850448.9
(22) Date of filing: 16.11.2006
(51) Int. Cl.: A61K 39/00

(54) **METHODS FOR INHIBITING CARCINOGENESIS AND/OR METASTASIS IN AN INDIVIDUAL WITH ENDOGENOUS C-MET LIGANDS AND INHIBITORS**
VERFAHREN ZUR HEMMUNG VON KREBSBILDUNG UND/ODER METASTASEN BEI EINER PERSON DURCH ENDOGENE C-MET-LIGANDEN UND -HEMMER
MÉTHODES DESTINÉES À INHIBER LA CARCINOGENÈSE ET/OU LA MÉTASTASE CHEZ UN INDIVIDU AU MOYEN DE LIGANDS ET D'INHIBITEURS DE C-MET ENDOGÈNES

(30) Priority: 16.11.2005 US 737331 P
(43) Date of publication of application: 20.08.2008
(73) Proprietor: Nayeri, Fariba, 582 16 Linköping (SE); Nayeri, Tayeb, 582 47 Linköping (SE)
(72) Inventor: Nayeri, Fariba, 582 16 Linköping (SE); Nayeri, Tayeb, 582 47 Linköping (SE)
(74) Representative: Rystedt, Per Hampus
(86) International application number: PCT/IB2006/003944
(87) International publication number: WO 2007/110698

(56) References cited:
- EP-A- 0 890 361
- WO-A-2005/016382
- US-A1- 2005 233 960
- SRIVASTAVA M ET AL: "ELEVATED SERUM HEPATOCYTE GROWTH FACTOR IN CHILDREN AND YOUNG ADULTS WITH INFLAMMATORY BOWEL DISEASE" JOURNAL OF PEDIATRIC GASTROENTEROLOGY AND NUTRITION, RAVEN PRESS, NEW YORK, NY, US, vol. 33, no. 5, November 2001 (2001-11), pages 548-553, XP008044741 ISSN: 0277-2116

## Description

### FIELD OF THE INVENTION

The present invention is based on the presence of endogenous native c-met ligands and inhibitors of individuals that inhibit the interaction and the function of variants of hepatocyte growth factor (HGF) that are produced autocrinally by transformed epithelial cells. The present invention is directed toward methods for inhibiting carcinogenesis and/or metastasis in an individual. The methods comprise administering to the individual an effective amount of endogenous c-met ligand or fragment thereof.

The present invention is also directed to methods for inhibiting the interaction of endogenous HGF produced autocrinally, methods for enhancing the effects of HGF produced endocrinally, methods for inhibiting the effects of autocrinally produced HGF, methods for enhancing the motogenic effect of HGF produced by mesenchymal cells, and methods for affecting angiogenesis, cell division, migration and/or morphogenesis in an individual. The present invention is further directed to methods of regulating the amount of endogenous c-met ligand or fragment thereof in an individual and for distinguishing between autocrine and endocrine variants of HGF.

### BACKGROUND OF THE INVENTION

The *met* oncogene was originally identified in a chemical carcinogen-treated (*N*-methyl-*N*'-nitrosoguanidine) human osteogenic sarcoma cell line (MNNG-HOS). *Tpr-met* was isolated from these cells as a gene which could morphologically transform murine NIH 3T3 fibroblast cells in vitro and induce them to form tumors in athymic nude mice. The *tpr-met* gene represents the fusion of two distinct genetic loci: *tpr* and *met.* Although the (*tpr*) oncogene product is a cytosolic kinase, the (*met*) proto-oncogene product is a transmembrane receptor-like protein. *C-met* is the proto-oncogene product, a receptor-like tyrosine kinase comprised of disulphide-linked subunits of 50 kD (α) and 145 kD (β). In the fully processed *c-met* product, the α subunit is extracellular and is heavily glycosylated, and the β subunit has extracellular, transmembrane and tyrosine kinase domains as well as sites of tyrosine phosphorylation.

Until 1990 *c-met* had no known ligands. Evidence that HGF induces rapid tyrosine phosphorylation of proteins in intact target cells suggested that tyrosine kinase receptor might mediate its mitogenic signal. A 145 kDa tyrosil phosphorylation was observed in rapid response to HGF treatment of intact target cells and was identified as the β subunit of *c-met.* ¹²⁵I -Labelled ligand to cellular proteins of appropriate size that are recognized by antibodies to *c-met* established the *c-met* product as the cell-surface receptor for HGF. It is the presence of the intracellular tyrosine kinase domain that groups the HGF receptor as a member of the receptor tyrosine kinase (RTK) family of cell surface molecules. The *c-met* receptor is exposed in the basolateral plasmalemma and is associated with detergent-insoluble components in polarized cells. The *met* gene has been mapped to chromosome 7 at q21-31 and. encodes a 1408 amino acid glycoprotein which lacks significant homology to any other known growth factor receptors.

Interaction of the c-met receptor with its ligand, HGF, induces signals via stimulation of the tyrosine kinase activity of the receptor, which in turn phosphorylates and promotes binding to the activated receptor of a number of intracellular signaling proteins. HGF promotes the growth and scattering of various cells. Coexpression of HGF and the *c-met* receptor in the same cell is observed in some cancer cell lines, contributing to autocrine regulation of cell invasion and tumorigenicity.

### SUMMARY OF THE INVENTION

Embodiments of the present invention are directed towards methods for inhibiting carcinogenesis and/or metastasis in an individual. The methods comprise administering to the individual an effective amount of anti c-met immunoglobulin. In embodiments of the present invention, the endogenous c-met ligand or fragment thereof comprises endogenous anti c-met immunoglobulin, native and/or recombinant; endogenous biological active Hepatocyte Growth Factor (HGF), native and/or recombinant, and/or a combination thereof.

Embodiments of the present invention are also directed towards methods for inhibiting the interaction of endogenous HGF produced autocrinally to the *c-met* receptor in an individual. The methods comprise administering to the individual an effective amount of endogenous c-met ligand or fragment thereof.

Embodiments of the present invention are also directed toward methods for affecting angiogenesis, cell division, migration and/or morphogenesis in an individual. The methods comprise administering to the individual an effective amount of endogenous c-met ligand or fragment thereof, anti-HGF or fragment thereof, or a combination thereof.

Embodiments of the present invention are further directed toward methods for regulating the amount of endogenous c-met ligand or fragment thereof in an individual. The methods comprise detecting the level of endogenous c-met ligand or fragment thereof in the individual and administering an effective amount of endogenous c-met ligand or fragment thereof to the individual.

### DETAILED DESCRIPTION OF THE FIGURES

The following Detailed Description may be more fully understood in view of the figures, in which:

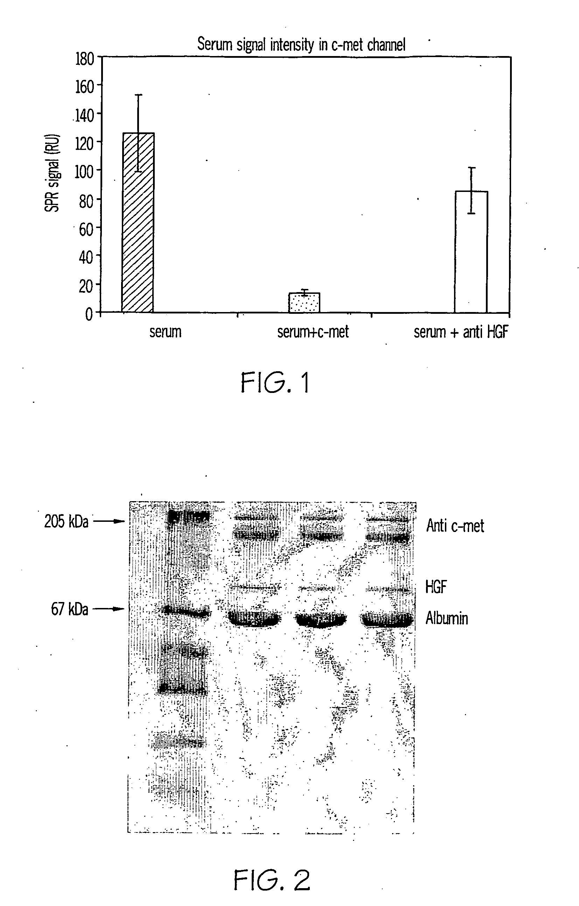

**Figure 1****:** Histogram showing the signal intensity in the flow cell immobilized by c-met comparing serum (n=10), before, after addition of commercial anti c-met antibodies (p<0.00001) and after addition of commercial anti-HGF antibodies (p<0.001). There is a difference between the reduction of signal after anti c-met and anti HGF addition;
**Figure 2****:** SDS-page of serum from 3 blood donors after purification in affinity column immobilized by c-met. The eluted sample is ultra-filtered. The concentration of acrylamide in separating gel is 11%. The high molecular bands belong to anti c-met;

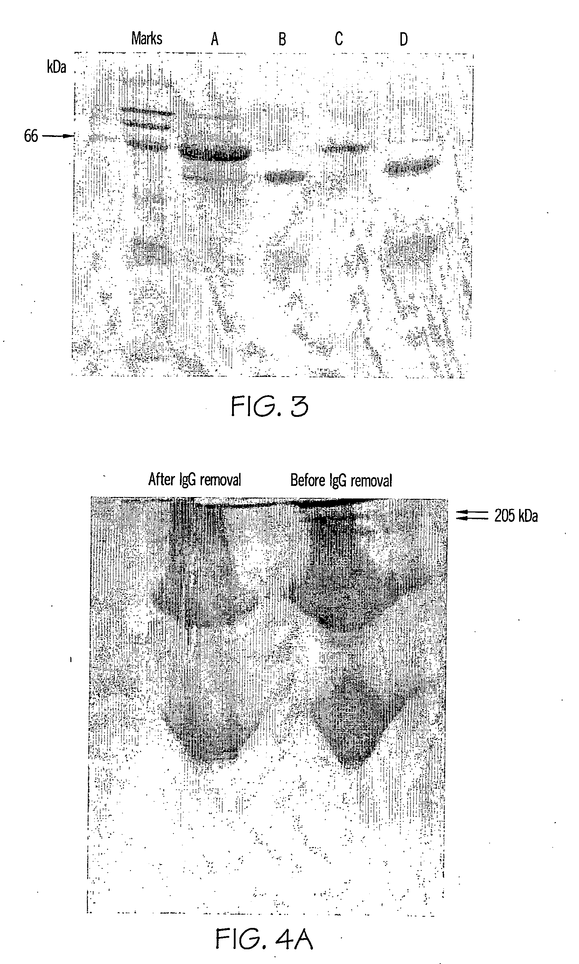

**Figure 3** SDS-page of serum sample (blood donor) after purification in affinity column immobilized by c-met. The concentration of acrylamide in separating gel is 11 %.; A- Sample of blood donor purified with c-met column and ultra filtered, for first time; B- Sample of A after albumin and IgG removal; C- Sample of blood donor purified with c-met column and ultra filtered, for second time; and D - Sample of C after albumin and IgG removal;

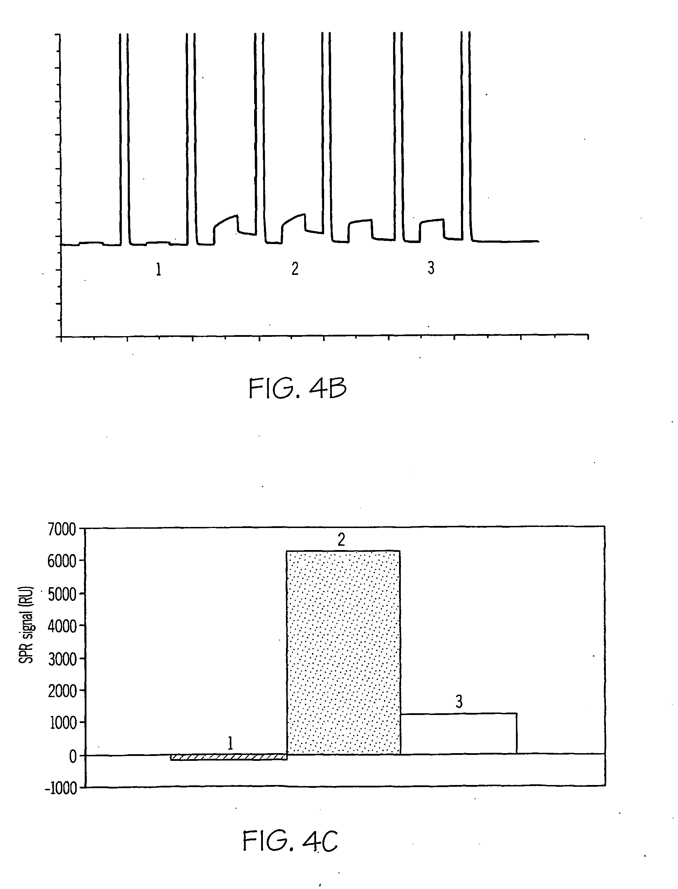

**Figure 4****:** A: SDS-page analysis of human hepatitis Hbs immunoglobulin (Aunativ) before and after albumin and IgG removal. The two high molecular bands disappear after IgG removal. The concentration of acrylamide in separating gel is 14.67%; B: The SPR flow cell immobilized by c-met curve. The signal 1 is the signal after injection of commercial c-met recombinant. Signal 2 belongs to Aunativ and signal 3 belongs to Aunativ after albumin and IgG removal. Each sample is tested twice; C: Sensogram demonstrating differences between the signal intensity (1-2-3);

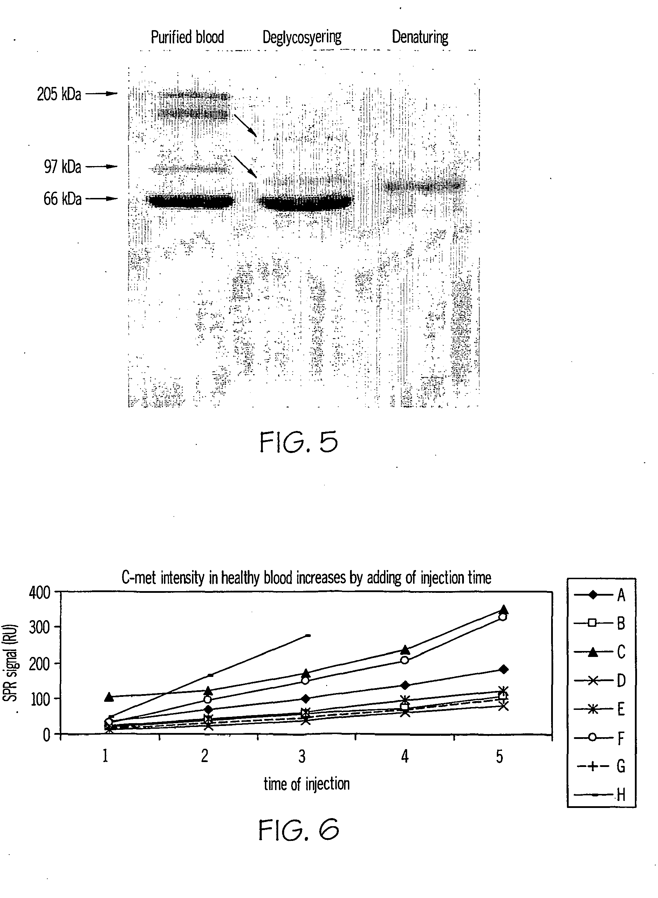

**Figure 5****:** SDS-page of serum sample (blood donor) after purification in affinity column immobilized by c-met. The concentration of acrylamide in separating gel is 11 %. There is a decrease in the molecular weight of anti c-met (>120 to 100 kDa) and HGF (95 to 75 kDa) after deglycosylering;
**Figure 6****:** A diagram of the signal intensity in SPR flow cell immobilized by c-met in serum from well-trained volunteers. The *signal intensity increases* by adding the time of sample injection (1-3-5-7-10 minutes);

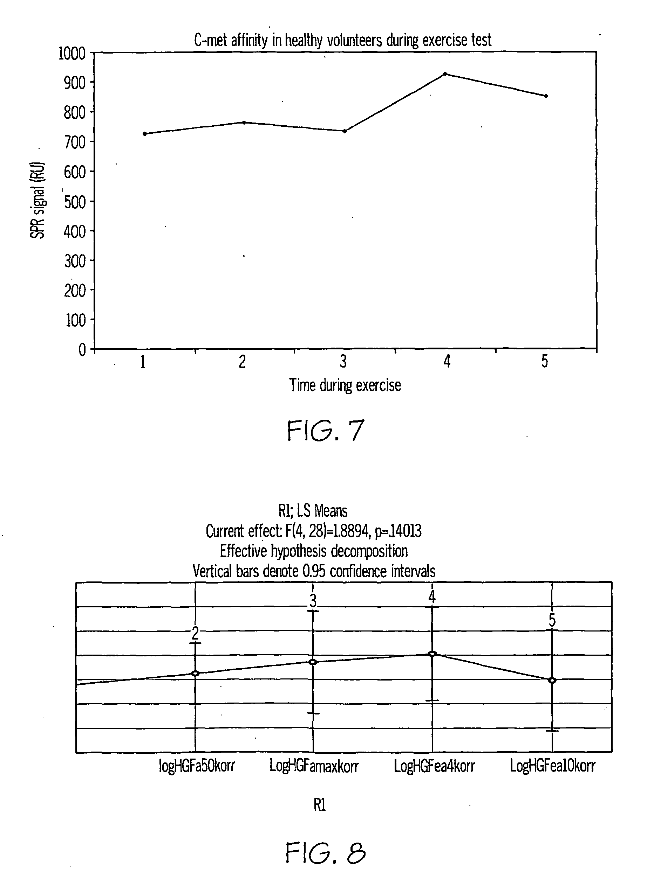

**Figure 7****:** A graph showing the mean signal intensity in a surface plasma resonance (SPR) flow cell immobilized by c-met in serum from 8 well-trained volunteers increased non-significantly during exercise in all of the cases. 1=before exercise, 2= at half exercise, 3=at peak exercise, 4= four and 5= ten minutes after exercise;
**Figure 8****:** A graph showing the mean plasma concentration of HGF from 8 well-trained volunteers increased non-significantly during exercise in all of the cases. 1=before exercise, 2= at half exercise, 3=at peak exercise, 4= four and 5= ten minutes after exercise;

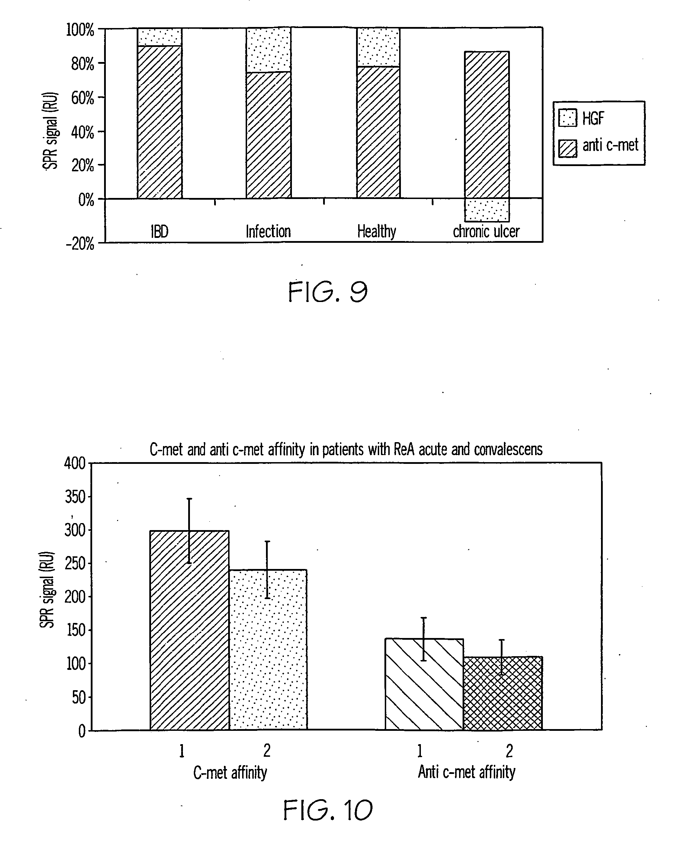

**Figure 9****:** Histogram showing the percent of SPR signal intensity in channels immobilized by c-met and anti HGF antibodies, reflecting anti c-met and HGF present in blood respectively, in different groups with IBD (n=48), infectious gastroenteritis (n=9), healthy control (n=9) and chronic leg ulcers (n=9). In the patients with chronic leg ulcers there is negative SPR signals in affinity to anti-HGF antibodies;
**Figure 10****:** Histogram showing the SPR signal affinity in patients with reactive arthritis (ReA) at the acute stage of disease (1) and after 3-6 months (2) in both flow cells immobilized by commercial c-met and anti c-met. The affinity to c-met flow cell (c-met channel) reflects presence of anti c-met in blood and the affinity to anti c-met flow cell (anti c-met channel) reflects the presence of circulating c-met in blood. The levels of c-met and anti c-met decreases significantly from acute phase to the convalescence (p<0.01);

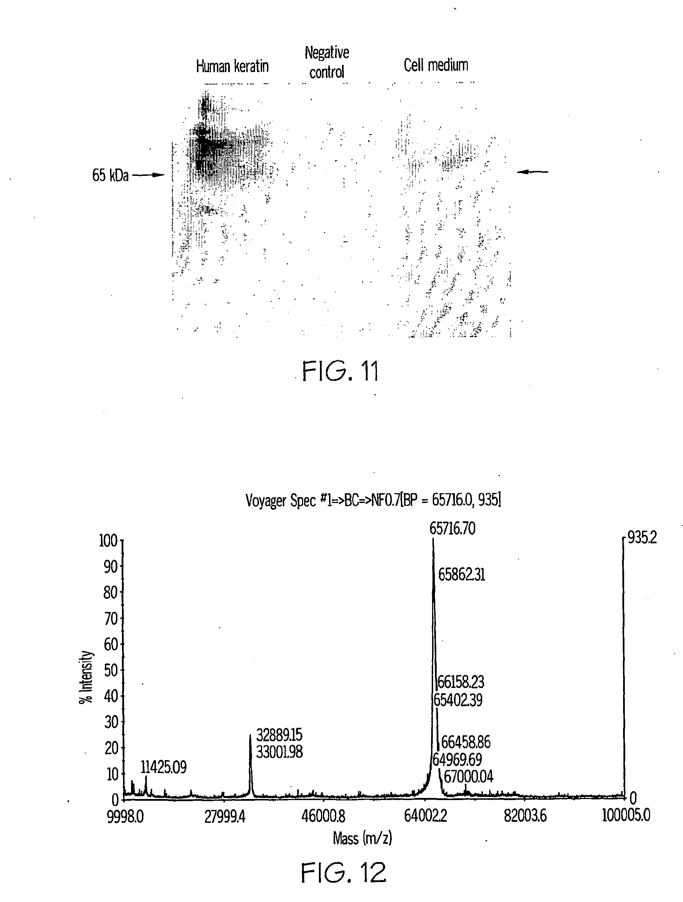

**Figure 11****:** Western blot analysis of medium obtained from culture of injured keratinocytes. Human keratin is used as positive control and bovine carbonic dehydrase as negative control.
**Figure 12****:** Matrix-assisted Laser desorption/Ionisation (MALDI-TOF) analysis of cell culture medium from skin keratinocytes of injured patient after purification in affinity column immobilized by c-met and ultra-filtration and albumin and IgG removal. Signals at about 66 and 33 kDa might belong to HGF chain;

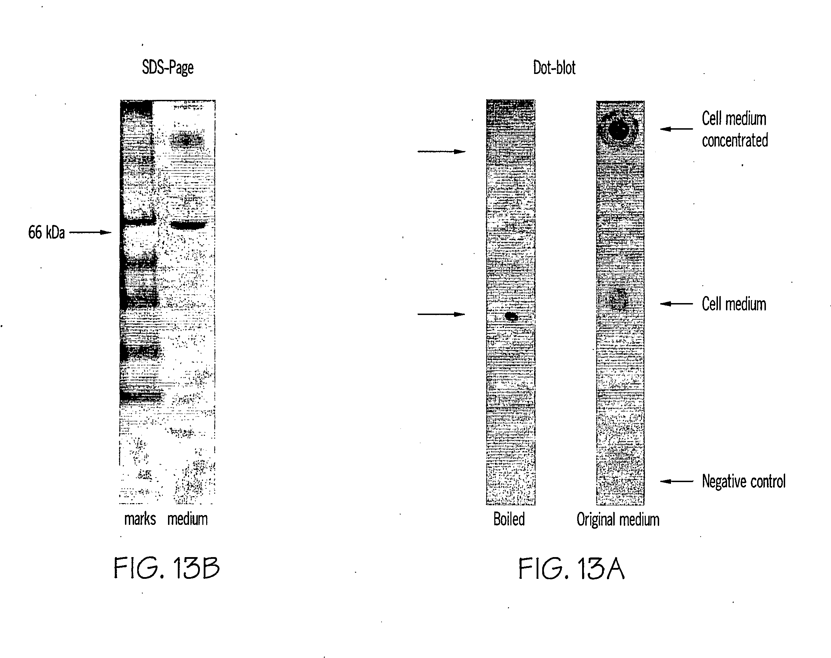

**Figure 13****:** A: Dot-blot analysis of cell culture medium of skin keratinocytes from the injured patient. The dot-blot analysis compares the results from cell culture medium before (original medium) and after (boiled) boiling of sample; B: SDS-Page of medium of skin keratinocytes from the injured patient. The concentration of acrylamide in separating gel is 14.67%;

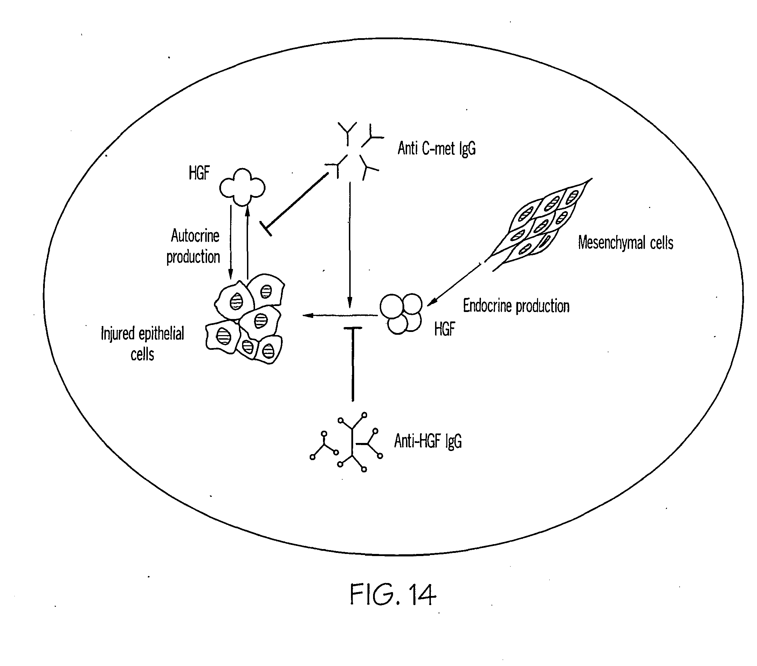

**Figure 14****:** Illustration of the two ways of producing HGF. Mesenchymal cells produce HGF in an endocrine fashion as a response to injuries in the body. This HGF has effects on epithelial cells that are inhibited by anti-HGF antibodies. Native anti c-met induces such an effect on epithelial cells. The injured epithelial cells might produce HGF autocrinally that has effect on the same cells and induces cell division and migration. Native anti c-met antibodies inhibits this effect;

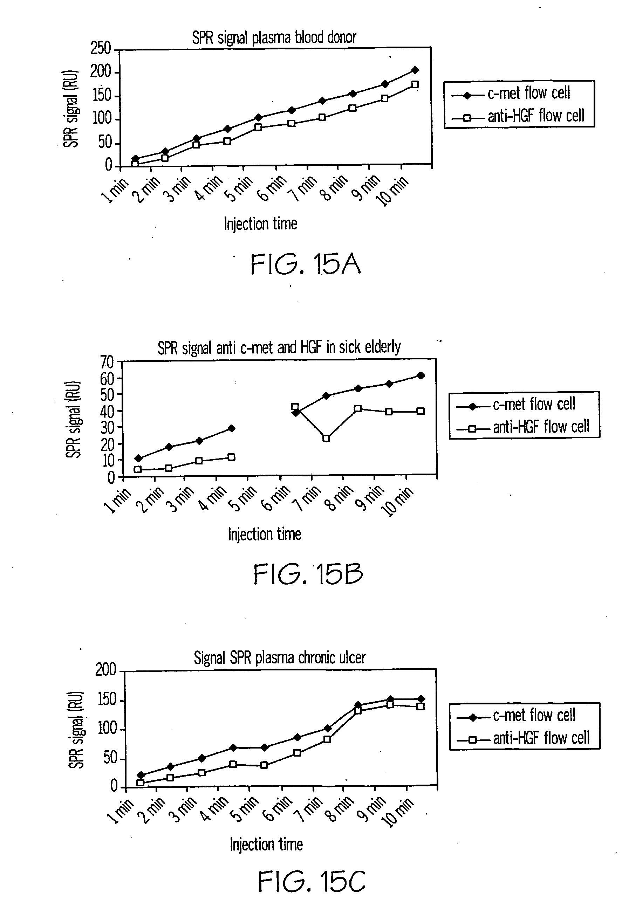

**Figure 15****:** A: A graph showing the SPR signal intensity in flow cells immobilized by c-met and anti HGF antibody in a healthy blood donor with relatively low signal intensity in c-met channel. The time of injection is increased from 1-10 minutes. The affinity to channels increases by adding the time of injection (blood full loaded by anti c-met and HGF), B: A graph showing the SPR signal affinity for c-met and anti HGF flow cells, in a very sick old patient. The maximum affinity to flow cells is much lower than healthy control. The anti HGF affinity (circulatory HGF) does not follow c-met affinity; C: A graph showing the SPR signal affinity for c-met and anti HGF in 49 years old patient with chronic leg ulcer who had the same signal intensity in c-met channel as the healthy blood donor. The affinity to both c-met and anti HGF flow cells stop to increase after 7 minutes of injection; and

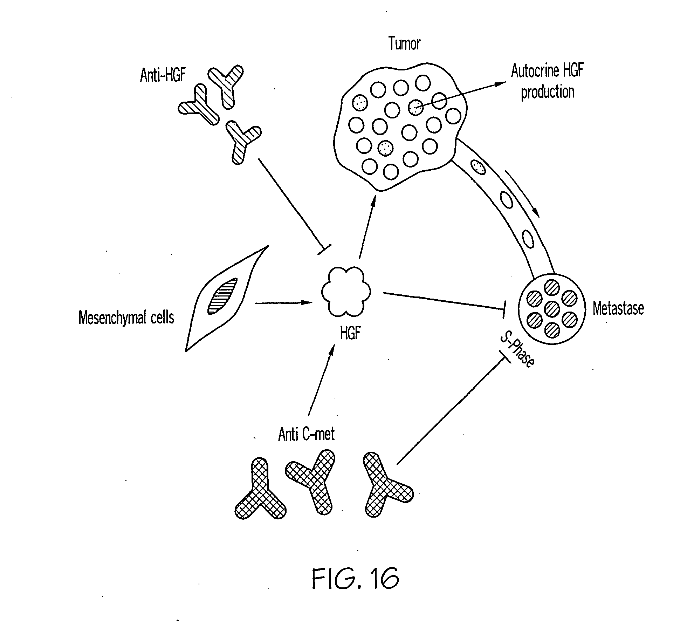

**Figure 16****:** Illustration depicting that cancer tumors contain two kinds of cells; cells expressing C-met/HGF receptor and cells expressing both *c-met* receptor and HGF mRNA. The latest is high invasive cancer cells that both produce and have effect of HGF (autocrine loop). The autocrine HGF producing cells invade in the blood stream and cause metastasis. Mesenchymal cells produce normal HGF (endocrine loop) that has positive effect on injured normal epithelial cells. This HGF might induce cell division and migration of tumor cells that express HGF receptor but this effect is inhibited completely by anti-HGF produced in the body as a regulatory mechanism. Anti-HGF on the other hand can not inhibit the effects of HGF produced autocrinally by the cells. These cells might invade and cause metastasis in the other organ. Addition of HGF produced by mesenchymal cells (endocrine loop) or anti c-met, at the S-phase, can inhibit the growth of the high invasive cells. Anti c-met might as well inhibit the motogenic effect of HGF produced by these cells. Native anti c-met do not inhibit the effects of normal HGF (endocrine loop).

### DETAILED DESCRIPTION OF THE PREFERRED EMBODIMENTS

Hepatocyte Growth Factor (HGF) is a multifunctional cytokine that is produced during injuries in the body. The inventors have determined that amounts of HGF increase during infection in blood as well as in the injured organ. The amounts of HGF decrease as soon as the appropriate treatment is initiated. HGF is quite stable in blood and body fluids. The interaction of HGF with its high affinity receptor, c-met, induces signals via stimulation of tyrosine kinase activity that result in mitogen, motogen and morphogen effects of HGF and initiate sequences of events in the cell that result in regeneration of cells and organ after injuries.

The inventors have studied the concentration and stability of HGF in blood and observe that the mean and median concentration of HGF is about 0.7ng/mL in healthy blood donors. Further the inventors have studied recombinant HGF and in body fluids with Surface Plasmon Resonance (SPR) and show that biologically active HGF has constant affinity to flow cells immobilized by c-met, monoclonal/polyclonal anti HGF antibodies as well as dextran.

The SPR technique is suitable for studying biomolecular interactions on, or close to, a surface. SPR enables rapid detection in real time without any labeling of samples and can be utilized for concentration determination, kinetics studies and epitope mapping. In brief, light incident on a metal surface at a given angle of incidence can excite a surface-bound electromagnetic wave, a surface plasmon, which propagates along the interface between the metal and the ambient medium. Associated with the surface plasmon is an evanescent field that probes local changes in the refractive index of the ambient medium, induced, for example, by binding of a biomolecule to the surface. The change in refractive index will shift the angle of incidence at which the SPR excitation occurs. This shift in the angle is tracked by monitoring the movement of *intensity minima* of the reflected light with time using the Kretschmann configuration, and the binding event is presented as a sensorgram. The sensor surface of the SPR apparatus consists of a carboxy-methylated dextran matrix, which is a hydrogel providing a solution-like environment in which the biospecific interactions occur. The carboxyl groups in the dextran matrix enable covalent coupling of ligands (proteins, receptors, DNA etc) to the surface.

Using the SPR technique, the inventors have established a method to distinguish between infectious and non-infectious diarrhea. The inventors have also characterized the different properties of HGF found during infectious diseases with biologic activity and HGF found during some chronic diseases such as chronic ulcer. Using CCL-53.1 cells, transformed mouse skin epithelial cells, which express HGF receptor (c-met), the inventors have established a method for evaluation of HGF activity. The inventors have recently reported autocrine production of HGF from traumatized human skin epithelial cells.

While establishing a SPR method for detection of HGF in blood samples the inventors observe a high level of affinity to the flow cell immobilized by c-met that is several times more than affinity to monoclonal anti HGF antibodies or dextran in some cases. The inventors observe the same phenomenon while studying several blood samples from different patient groups. The inventors find no differences between paired serum and plasma samples. As the extracellular domain of recombinant c-met (R&D Systems) is fused to the carboxy terminal 6X histidine-tagged region of human IgG via a polypeptide linker, a false signal due to interaction of complement proteins with IgG is suspected. EDTA has an inhibitory effect on complement activation. Therefore, the inventors added different concentrations of EDTA to the samples, which do not have any effects on signal intensity in the flow cells. Based on the inventors' observations, the possible alternatives that might have-caused the signal are investigated.

C-met has been recognized as the cell surface receptor for HGF and while not wishing to be bound by theory, it is believed that no other proteins share the *c-met* receptor. Purification of blood samples in affinity column immobilized by c-met is used. The SDS-page of eluted sample after ultra-filtration revealed bands with high molecular weight and highly glycosylated that disappeared after albumin and IgG removal. At the same time the SPR signal at the flow cell immobilized by c-met (c-met channel) decreases. It is suggested that an anti c-met immunoglobulin in the blood samples causes the high affinity signals. Further investigations show that the SPR signal is decreased slightly after addition of anti-HGF antibodies to the samples and this signal reduction is proportional to the affinity signals in the flow cells immobilized by anti HGF antibodies (data not shown). The signal highly decreases after addition of c-met and increases after addition of anti c-met antibodies to the samples prior to analysis that might suggest that the affinity signal to the c-met channel is due to affinity of both, HGF and anti c-met in the blood. The slight reduction of signal after addition of human albumin to blood samples may depend on the affinity of native, biologically active HGF in the healthy blood to albumin and inhibition of interaction of HGF with the receptor. Furthermore the inventors observe that high concentrated human hepatitis Hbs immunoglobulin (Aunativ, Octapharma), and in a lower degree human normal immunoglobulin (Gammanorm, Octapharma) cause similar signals at c-met channel that disappeared after albumin and IgG removal that is in agreement to the suggestion of presence of anti c-met in the blood. Further, the inventors have established ELISA method to determined the total amounts of endogenous anti HGF antibodies (IgG and IgM) in the blood.

In order to determine the possible role of anti c-met in the blood, the inventors investigate the samples of healthy as well as different patient groups. Although there is no significant correlation to the age of patients, the inventors observe that the young and healthy as well as elderly that exercise regularly have high signal levels of anti c-met. Four patients with immunodeficiency (total IgG deficiency, data not shown) and the elderly and severe ill patients have very low or no signals at the c-met channel (Figure 15). The blood samples from 8 well-trained firemen are analyzed and show that the signal intensity increases by adding the time of injection from 1-10 minutes. During exercise test the signal intensity increases to maximum 4 minutes after the peak exercise but the difference did not reach significance due to few cases that are included in the study. The concentration (ELISA) and affinity to anti HGF flow cell (SPR) increases in the same manner. While not wishing to be bound by theory, this may suggest that the concentration of HGF, its activity, as well as anti c-met increases during exercise.

The patients with IBD have low signal affinity to c-met channel compared to acute gastroenteritis and healthy controls. Although the difference between signal affinity in the group with chronic leg ulcers and healthy control did not reach significance (p<0.053), the inventors observe that the elderly patients with low activity and/or on corticosteroid treatment had much lower levels of anti c-met compared to younger and active patients. Remarkably the inventors observe that the signal affinity in flow cells immobilized by anti-HGF antibody is significantly lower in the patients with IBD and chronic leg ulcers compared to healthy controls. The inventors have previously shown that the affinity of biologic inactive HGF decreased significantly to anti-HGF antibodies, heparan sulphate proteoglycan (HSPG) and dextran sulfate. While not wishing to be bound by theory, this observation may suggest that in the patients with IBD and chronic leg ulcers, the biological activity of HGF decreased both in serum and locally in bowel respective in ulcers.

The inventors have previously shown that HGF concentration increases during infection. The patients with pneumonia in the study have high levels of HGF (ELISA) that decreases with efficient antibiotic therapy but continues to increase in the case of therapy failure. This is not the case in SPR signal intensity in c-met channel. The signal decreases significantly during disease, which may indicate consumption of anti c-met during infection.

In the patients with ReA the signal intensity at anti c-met channel is high at the abrupt of disease which might indicate cell dead during acute inflammation and release of cell binding receptor, c-met in the blood (circulatory c-met). During the convalescence the c-met and anti c-met levels in blood decreases significantly which might suggest the regulatory effect of anti c-met during inflammation in the body. The presence of circulatory c-met and overexpression of circulating c-met and its correlation to early recurrence in non-small cell lung cancer has been recently reported.

Enhanced expression of c-met receptor in cancer cells has been reported since 1990. The c-met receptor encoded by the Met oncogene controls a genetic program, known as "invasive growth", responsible for several developmental processes and involved in cancer invasion and metastasis. Invasive growth is defined as a complex biological program which instructs cells to dissociate, migrate, degrade the surrounding matrix, proliferate and survive. Individual aspects of this process can be controlled by many cytokines and growth factors. However, coordinated regulation of invasive growth as a whole is specifically accomplished by HGF, which acts through c-met receptor. Review of the most recent studies shows that an overexpression of c-met and its correlation to worse survival is suggested in several malignant tumors such as chordomas, advanced epithelial ovarian cancer, transitional-cell carcinoma of the bladder, malign melanoma, human colorectal cancer, endometrial carcinomas, B-cell lymphoma and node-positive breast cancer. The coexpression of HGF and c-met in epithelial cells mediates autocrine HGF-met signaling. The autocrine signaling of HGF and its role in proliferation and migration of cancer cells has been studied in SNU-484 gastric cancer cell line, meningiomas, malign fibrous histiocytoma, breast carcinoma, ovarian carcinogenesis, colorectal cancer, synovial sarcoma, bone and soft tissue tumors, activated monocytes and HGL4 glioblastoma cells. There are in-build mechanisms of negative feedback regulation in the body, such as transcriptional induction of Notch function, which in turn limits HGF activity through regression of the c-met.

Human keratinocytes are epithelial cells and not supposed to produce HGF. The skin cells obtained from the injured patient are keratinocytes because they produced keratin. Thus the cells produced HGF in an autocrine fashion. The produced HGF has the properties of a biologically active HGF defined by SPR (submitted article), has two bands representing α and β chain as defined by MALDI-TOF and induces migration of CCL-53.1 cells. A similar observation that gastric fibroblast-conditioned medium have similar effects as HGF on epithelial proliferation and restitution and its activity is neutralized by anti-HGF antibody has been reported. However on the contrary to the HGF produced in the healthy or recombinant, the migration of CCL-53.1 cells produced by injured keratinocytes is not inhibited by anti HGF antibodies. Addition of commercial anti c-met or the native anti c-met, found in human immunoglobulin, inhibits the migration completely. The reason of this difference between HGF produced by mesenchymal cells and HGF produced in an autocrine fashion by epithelial cells is not known. However it might be of value to notice that anti c-met does not inhibit the effect of normal active HGF (in blood, body fluids or recombinant) on the CCL-53.1 cells. The inventors determined that the motogenic activity in CCL-53.1 cells increases by addition of anti c-met that functioned as an activator (data not shown).

Native, biologically active HGF and anti c-met are added to normal keratinocytes and *autocrine* HGF producing keratinocytes at the time of passage. Neither HGF nor anti c-met has effect on normal keratinocytes. However both HGF and anti c-met cause detachment of cells from the bottom of wells and cell dead in HGF producing epithelial cells (autocrine). A similar observation about HGF inhibition of growth of hepatocellular carcinoma cells that produced HGF autocrinally has been reported. Further the inventors have studied the MDA-MB-157 (HTB-24 ATCC) cell line, a human breast cancer cell line that autocrinally produces HGF, and observe that endogenous anti c-met added at the time of passage to cells causes cell death in HTB cells that confirms the previous results. Addition of cell culture medium from HTB-24 cells to injured CCL-53.1 cells causes migration of cells towards injured area. This effect is inhibited by addition of endogenous c-met inhibitor.

Cancer tumors contain two kinds of cells; cells expressing c-met/HGF receptor and cells expressing both c-met receptor and HGF mRNA. The latest is high invasive cancer cells that both produce and are affected by HGF (autocrine loop). The autocrine HGF producing cells (transformed epithelial cells) migrate in the blood stream and cause metastasis. Mesenchymal cells produce normal HGF (endocrine loop) that has regenerative effect on injured normal epithelial cells. This HGF may induce cell division and migration of tumor cells that express HGF receptor but this effect is inhibited completely by anti-HGF produced in the body as a regulatory mechanism. Anti-HGF on the other hand can not inhibit the effects of HGF produced by transformed epithelial cells. These cells may invade and cause metastasis in the other organs. The two forms of HGF (endocrine or autocrine produced) might differ pre- or post-transcriptionaly, in the configuration or glycosylation. Addition of HGF produced by mesenchymal cells (endocrine loop) and/or anti c-met, at the S-phase, inhibit the growth of transformed epithelial cells. Anti c-met might as well inhibit the motogenic effect of HGF produced by these cells.

The inventors have determined methods for inhibiting carcinogenesis and/or metastasis in an individual comprising administering to the individual an effective amount of endogenous c-met ligand or fragment thereof. One skilled in the art will appreciate the various methods for administering an effective amount of endogenous c-met ligand or fragment thereof to the individual, any of which may be employed herein. One skilled in the art will also appreciate that the endogenous c-met ligand administered to the individual may comprise full-length endogenous c-met ligand, a endogenous c-met ligand fragment or a combination thereof. As used herein, "endogenous c-met ligand fragment" refers to any fragment of the full-length endogenous c-met ligand that has the same activity as full-length endogenous c-met ligand, or is a chemical or structural analogue of the full-length endogenous c-met ligand. Examples of endogenous c-met ligands include, but are not limited to, native and/or recombinant anti c-met immunoglobulin, native and/or recombinant Hepatocyte Growth Factor (HGF), or a combination thereof.

The inventors have also determined methods for inhibiting the interaction of endogenous HGF produced autocrinally to the c-met receptor in an individual. The methods comprise administering to the individual an effective amount of endogenous c-met ligand or fragment thereof. Such inhibition of the interaction of endogenous HGF produced autocrinally will also enhance the interaction of endogenous HGF products endocrinally to the c-met receptor.

Anti c-met does not inhibit the effects of normal HGF (endocrine loop) (Figure 16) and its production may be enhanced by exercise and consumed during severe inflammation. Thus training might trigger healing of damages and inhibit cancer invasion mediated by HGF/c-met interaction. Angiogenesis, cell division, migration and morphogenesis are affected by endogenous c-met ligand, such as HGF, c-met, native anti c-met and anti-HGF. Accordingly, the inventors have determined methods for affecting angiogenesis, cell division, migration and/or morphogenesis in an individual. The methods comprises administering to the individual an effective amount of endogenous c-met ligand or fragment thereof, anti-HGF or fragment thereof, or a combination thereof.

The regenerative effects of anti c-met in combination to endocrinally produced HGF on injured epithelial cells and the apoptotic effect on transformed epithelial cells, might motivate induction of production or exogenous administration of anti c-met. Exercise and vaccination are examples to induce native anti c-met production. Exogenous administration of endogenous c-met ligand or fragment thereof before, during, after surgery and/or after the tumor is removed might abolish the high invasive transformed epithelial cells and decrease the risk of distant metastasis. The high sensitive, high specific Nanotechnology might distinguish and abolish specifically the transformed epithelial cells that express HGF mRNA in tumors to reduce the risk of invasion. A similar phenomenon of presence of different kinds of a growth factor with different properties on normal and transformed cells might be true in the case of other cytokines.

The inventors have determined that native anti c-met antibodies are found in large amounts in blood of healthy human beings. The antibody is a high glycosylated immunoglobulin that is consumed during acute or chronic inflammation and might increase by physical training and thus, the production of antibody might be enhanced by exercise and consumed during inflammation. The present invention may also be directed to determining the presence of low amounts of anti c-met ligand in blood of patients with autoimmune diseases.

The inventors have further determined methods for regulating the amount of endogenous c-met ligand or fragment thereof in an individual. The methods comprise detecting the level of endogenous c-met ligand or fragment thereof in the individual and administering an effective amount of endogenous c-met ligand or fragment thereof to the individual.

### Examples

The experimental work is described in detail:

### Study subjects

### A- Healthy volunteers

Serum is collected from 46 healthy blood donors (22 women, 24 men; 18-57 years old, median 36 years old).

Eight healthy well-trained volunteers (6 men, 2 women; mean age 36, range 25-49 years) are also included in the study. In these eight volunteers exercise test is performed on an electrically braked *computer controlled* bicycle. Venous blood samples are drawn immediately before exercise (supine), at approximately half time at exercise, at peak exercise, 4 and 10 minutes after exercise (supine). The samples are kept at-70°C prior to analysis.

### B- Patients with pneumonia

Plasma is collected from 16 patients (10 men and 6 women; 22-90 yeas old, median 72) with x-ray verified pneumonia before and after 18-24 hours of antibiotic treatment and kept at -70°C prior to analysis. These patients have been included as a part of previous study (Accepted article Chemotherapy)

### C- Patients with Inflammatory bowel disease (IBD) and Infectious gasteroenteritis

Plasma is collected from 48 patients (28 women and 20 men; range 19-85, median 56 years old) with a known IBD that are controlled at the Department of Gastroenterology, University Hospital in Linköping.

Plasma is also collected from 9 patients (6 women, 3 men; range 25-86, median 65 years old) who are admitted to the Department of Infectious Diseases with acute gastroenteritis. Feces cultures are positive for *Campylobacter jejuni* in 2, *Clostridium deficile* in 2, Salmonella DO in 2 cases and are negative in 3 cases. These patients have been included as a part of previous study.

### D- Patients with chronic leg ulcers

Blood is collected from 12 patients with chronic leg ulcers (8 women, 4 men; 44-89 years of age, Median 76 years old). These patients have been included as a part of previous study.

### E- Patients with Reactive Arthritis

Blood is collected from 10 patients (7 women, 3 men; range 17-78 years old, Median 36 years old) with reactive arthritis, developed after infectious gastroenteritis, at the acute stage of disease and after 3-6 months.

### F- Patients with Wegeners Granulomatous Vasculitis

Blood is collected from 11 unidentified patients with a know Wegeners Granulomatous Vasculitis.

### Methods

### Ligand immobilization procedures for SPR measurements

SPR measurements are conducted at 760 nm in a fully automatic Biacore 2000 instrument (Biacore AB, Uppsala, Sweden) equipped with four flow cells. The flow cell temperature is 25 °C in all experiments. The sample surfaces used are carboxy-methylated dextran CM5 chips (Biacore AB, Uppsala, Sweden). Coupling of ligands to the carboxylic acid groups of the dextran hydrogel is carried out by conventional carbodiimide chemistry using 200 mM EDC (N-ethyl-N'-(3-diethylaminopropyl) carbodiimide) and 50 mM NHS (N-hydroxysuccinimide). The activation time is 7 min, followed by a 2-7 min ligand injection. Deactivation of remaining active esters is performed by a 7 min injection of ethanolamine/hydrochloride at pH 8.5. A flow rate of 5 µl//min is used during immobilization. The ligands are diluted in 10 mM acetate buffer pH 4.5, i.e., below the protein isoelectric point, thus enhancing the electrostatic interactions between the dextran matrix and the ligands. The recombinant Met proto-oncogene receptor (100 µg, R&D Systems), monoclonal anti HGF receptor (100µg/ml, R&D Systems) and human albumin 200mg/mL (Octaphanna) are diluted 1:5. The contact time is 2-7 minutes. The samples are diluted in HBS buffer, PBS (pH 7.4), MQ water, Physiologic Nacl + EDTA in different concentrations and different combinations and tested separately. To obtain reliable results a maximum of 80 tests are done in one chip. Burate (pH 8.5) and/or a combination of 5 mM glycine buffer pH 2.0 and 1 M NaCl are used as regeneration buffers.

### Purification of blood samples by affinity chromatography and SDS-PAGE

The affinity chromatography columns (Hi-trap Amersham Biosciences) are immobilized with recombinant human HGF receptor (c-MET)/Fc chimera (R&D Systems). Serum or plasma samples from blood donors are reconstituted in PBS (pH 7.4) and injected to the column. 5 mM glycine buffer pH 2.0 with 1 M NaCl was used as elution buffer. The samples are then applied to the 500 µl centrifugal tubes (Amicon Ultra, Millipore, S.A.S. Molsheim, France) and centrifuged at 4000 G for one hour. Albumin and IgG removal kit (Amersham Biosciences) and native deglycosylation kit (Sigma Alrich) using the manufacturers instruction are utilized

SDS-PAGE is performed with the blood samples before and after purification, filtration and albumin and IgG removal, using a 4% stacking gel and a 11-14.6% running acrylamide gel. For size estimation pre-stained protein standard (Sigma Aldrich) is electrophoresed simultaneously.

### Primary keratinocyte culture

The cells obtained from skin biopsy at the inguinal area of a 30-year-old male patient who suffered bum affecting 80% total body surface area, are seeded for tissue regeneration (Karocell AB Stockholm). The prepared cells are recultured in Kaighn's modification of Ham's F-12K medium (ATCC) supplemented with 15% horse serum, 2.5% fetal bovine serum (Sigma-Aldrich Stockholm, Sweden) and antibiotics (10%, penicillin, streptomycin, amphotericin B, Sigma Aldrich), in an atmosphere of 5% CO₂ and 95% air at 37°C. A monolayer containing skin fibroblasts surrounding islands of keratinocytes is grown. Using sterile needles, collecting cells from keratinocyte-culture and re-culturing in the supplemented Ham's F-12K medium (as described above) the inventors succeeded to obtain a pure keratinocytes monolayer (producing keratin, article under preparation) after several passages. The medium is collected every fifth day, centrifuged 1000 g in 10 minutes and kept at - 70 C°. At the same time the confluent cell culture is separated with non-enzymatic cell dissociation solution (1x) (Sigma-Aldrich) and suspended in F-12K medium with 15% horse serum and 2.5% fetal bovine serum + antibiotics. Skin keratinocyte from healthy donors is obtained commercially (Karocell AB Stockholm).

CCL-53.1 adherent epithelial cells derived from mouse skin, melanocytes, melanoma are obtained from American Tissue Cell Collection (ATCC, Manassas, Virginia, USA) and are grown in Kaighn's modification of Ham's F-12K medium (ATCC) supplemented with 15% horse serum and 2.5% fetal bovine serum (Sigma-Aldrich) and 10% antibiotics, in an atmosphere of 5% CO₂ and 95% air at 37°C and inoculated on a 24-well culture plate (Nunc Brand Products, Roskilde, Denmark) and cultured under the same conditions for 24 hours until they reached confluence. The confluent monolayer is scraped by a sterile steel device. Detached cells *are* washed with PBS. The area of the square not covered by cells (mm²) is measured by microscope (Olympus) and documented in each well. The collected medium from cultivated human keratinocytes (1 ml) is then added to the cells and incubated at 37°C in a humidified atmosphere containing 5% CO₂. Medium collected from a previous passage of CCL-53.1 cells is used as negative control. SPR responsive recombinant human HGF (submitted article) and HGF purified from healthy blood donors in heparin affinity column are used as positive controls. After 24 hours the area not covered by monolayer is measured again and documented. The experiments are performed with and without anti-human HGF antibodies (R&D Systems) and anti HGF receptor/ c-Met antibodies (R&D Systems). Six independent experiments are done.

### Statistics

The values are log-normally distributed and analyses of variance by groups (ANOVA, SPSS 13.1) and linear regression are, therefore, used after transforming the values into their natural logarithms. Non-parametric test, Wilcoxen Signed Ranks Test is also used to verify some results. A probability level of p<0.05 is considered statistically significant.

### Results

Results: Signals are found in the SPR flow cells immobilized by recombinant c-met. The signal decrease significantly after addition of c-met in blood prior to analysis (p<0.00001). Addition of anti human HGF mono/polyclonal antibodies or human albumin decrease the signal significantly as well (p<0.001). However addition of commercial anti c-met antibody increases the signal intensity (p<0.01). There is no correlation between age and sex of patients and intensity of signals. The signal increases during exercise but decreased during disease. There are no significant differences between serum and plasma samples. Purification of blood in affinity column immobilized by c-met gives two bands over 150 kDa by SDS-Page. Removal of albumin and IgG resulted in disappearing of bands in SDS-Page and signals in SPR. The process of deglycolysering decreases the band to about 120 kDa. The same results are seen by analysis of anti human hepatitis HBs immunoglobulin. Addition of anti hepatitis B HBs antibody as well as commercial anti c-met, inhibits the motogen effects of HGF produced in an autocrine manner by epithelial cells but does not inhibit the effects of HGF produced in healthy blood. Addition of anti c-met or SPR responsive recombinant HGF to the S-phase of autocrine HGF producing skin epithelial cells causes cell death but did not affect normal skin epithelial cells.

### Healthy blood donors and well-trained volunteers

***SPR**:* Signals are detected in the flow cells immobilized by recombinant human HGF receptor/ c-met (c-met channel). There are no significant differences between SPR signals in paired serum and plasma samples (p=0.56). The most reliable results are obtained when the samples are diluted in PBS (pH 7.4). Dilution of samples in Physiologic Nacl + EDTA does not influence the results. The signals decrease slightly but significantly after addition of monoclonal/polyclonal anti HGF antibodies (p<0.001). The signals highly decrease after addition of recombinant human HGF receptor/ c-met to the samples prior to analysis (p<0.0001) (Figure 1). Addition of human albumin decreases the signals slightly but significantly (p<0.01).

However addition of human anti c-met antibody to the samples increases the signal intensity (p<0.01). Treatment of blood samples by albumin and IgG removal cause a significant reduction of signals (p<0.0001). Analysis of human hepatitis Hbs antibody (Aunative, Octapharma) gives identical signals in the c-met channel. The signals disappear after addition of c-met to the sample or albumin and IgG removal. Addition of albumin after albumin and IgG removal to the sample (Aunativ) does not give any signals.

**SDS-Page:** The purified plasma in affinity chromatography column immobilized with c-met is analyzed by gel electrophoresis. Two lines with molecular weight >120 kDa, one line at about 95 kDa and the third line at 66 kDa are found. Another fourth line at about 35 kDa is also found (Figure 2)

The lines at >120 and 66 kDa disappear after albumin and IgG removal, and the two distinct bands at about 55 and 25 kDa are visualized (Figure 3). SDS-Page of Aunativ gives two bands at >120 kDa that disappear after albumin and IgG removal (Figure 4). Deglycosylering of purified blood samples in affinity column causes a reduction of molecular weight of bands to about 100 respective 75 kDa (Figure 5).

**Study subjects:** There is no correlation between age and sex of study subjects and SPR signals (p=0.44). The c-met affinity in healthy blood donors increases by increasing the injection time (*Figure 6*). The *c-met* affinity signals in well-trained volunteers show a non-significant trend with an average (range) of 726 RU (171-1536) before exercise, 762 RU (230-1696) at half exercise, 733 RU (295-1558) at peak exercise, 924 RU (303-2051) four and 851 RU (316-1997) ten minutes after exercise (Figure 7). The HGF concentration in blood is also determined by ELISA. Serum HGF is corrected to mean albumin concentration in order to account for possible changes in hematocrit.

The corrected HGF shows a non-significant trend with an average (range) of 0.55 (0.31-0.77) before exercise, 0.60 (0.36-0.77) at half exercise, 0.70 (0.32-1.16) at peak exercise, 0.74 (0.36-1.30) four and 0.60 (0.31-1.03) ten minutes after exercise (Figure 8).

### Patients with pneumonia

There are no significant *differences* between the signal intensity in c-met channel between healthy controls and the patients with pneumonia (n=16, p=0.37). The signal intensity decreases significantly during treatment in all of the cases (Wilcoxen signed ranks test, p<0.01) no matter if they had responded to the therapy or not.

### Patients with IBD/acute gastroenteritis

Although there are no significant differences between signal intensity in the c-met channel between acute gastroenteritis (n=9) and healthy controls (n=9, analyzed at the same time), the signal levels are significantly lower in the patients with IBD (n=48) compared to acute gastroenteritis and healthy controls (p=0.005). The affinity of blood to anti HGF channel in patients with IBD is significantly lower than normal controls (p<0.01) and there is a discrepancy between affinity to c-met channel and anti-HGF channel.

### Patients with chronic leg ulcers

Although the signal intensity in the c-met channel, in some of the patients with chronic leg ulcers, is low, the overall difference between these patients (n=12) and the healthy controls (n=19, analyzed at the same time) *does not reach significance* (p=0.053). The affinity of blood to anti HGF channel in patients with chronic ulcers is significantly lower than normal controls (p<0.0001) (Figure 9) and there is a discrepancy between affinity to c-met channel and anti-HGF channel in these patients.

### Patients with Reactive arthritis (ReA)

There is no significant differences in the c-met channel affinity between the samples obtained from patients with ReA (n=10) and healthy controls (n=10). However these patients have a high level of signal in the flow cell immobilized by anti c-met compared to controls (p<0.01). The signal intensity in both flow cells immobilized by c-met and anti c-met decreases significantly from acute phase of disease to convalescence within 3-6 months (Wilcoxen Signed Ranks Test, p<0.01) (Figure 10).

### Patients with Wegeners Granulamatous Vasculitis

Although the affinity to anti HGF flow cell is the same as normal healthy controls, the inventors observe a significant reduction in affinity to c-met flow cell in these patients (n=11, p<0.05) compared to healthy controls (n=30) determined in the same chip.

### Biological activity on injured human keratinocytes

Human keratinocytes obtained from person with major skin damage produce keratin (Figure 11) and HGF (Figure 12-13). The medium obtained from cells induced motogenic activity in CCL-53.1 cells. Such an effect is not seen while studying the medium from culture of healthy skin keratinocytes. The motogenic effect of HGF produced autocrinally by injured keratinocytes could not be inhibited by monoclonal/polyclonal antibodies against HGF, while addition of polyclonal anti c-met antibody as well as Aunativ inhibited the motogen activity of CCL-53.1 cells towards the injured area completely. However in the wells that are treated with biologically active recombinant HGF or HGF purified from healthy blood donors in heparin affinity column (positive controls), addition of anti c-met could not inhibit the motogenic activity of cells, but this effect is inhibited completely with anti-HGF antibodies (Figure 14).

### Cells at S-phase

*CCL-53.1 cells:* Addition of SPR-responsive recombinant HGF, HGF purified from blood donors or anti-HGF to the cells at *passage* had no significant effect on cells. Addition of anti c-met caused detachment of cells in 4/6 experiments.

***Normal human keratinocytes*:** Addition of SPR responsive HGF or anti c-met at passage had no effect on cells.

***Epithelial cells producing HGF autocrinally*:** Addition of SPR responsive recombinant HGF at passage caused a significant reduction of number and attachment of cells to the bottom of wells in all experiments. Addition of anti c-met caused detachment of cells from the bottom (14 hours) and cell dead (24 hours) but did not affect the number of cells.

The specific illustrations and embodiments described herein are exemplary only in nature and are not intended to be limiting of the invention defined by the claims.

## Claims

1. Endogenous c-met ligand or fragment thereof for use in a method for inhibiting carcinogenesis and/or metastasis in an individual, comprising administering to the individual an effective amount of said endogenous c-met ligand or fragment thereof, wherein the endogenous c-met ligand is a native or recombinant human anti c-met immunoglobulin.

2. Endogenous c-met ligand or fragment thereof according to claim 1, **characterized in that** the endogenous c-met ligand is a native human anti c-met immunoglobulin obtained from serum of a healthy individual or an immunoglobulin preparation.

3. Endogenous c-met ligand or fragment thereof according to claim 1, wherein said endogenous c-met ligand or fragment thereof inhibits autocrine HGF-producing cells in the individual, and optionally the endogenous c-met ligand does not inhibit endocrine HGF-producing cells in the individual.

4. Endogenous c-met ligand or fragment thereof according to claim 3, wherein the effects of endocrine HGF-producing cells in the individual are enhanced.

5. Endogenous c-met ligand or fragment thereof according to claim 1, for use in a method for inhibiting carcinogenesis and/or metastasis in an individual wherein said endogenous c-met ligand or fragment thereof is administered before, during and/or after surgery and/or after the tumour is removed.

6. Endogenous c-met ligand or fragment thereof according to claim 5, for use in a method for inhibiting carcinogenesis and/or metastasis in an individual, wherein said cancer therapy comprises surgery.

7. Endogenous c-met ligand for use in a method for inhibiting interaction of autocrine-produced HGF with the c-met receptor in an individual, comprising administering to the individual an effective amount of said endogenous c-met ligand, wherein the endogenous c-met ligand is a native or recombinant human anti c-met immunoglobulin.

8. Endogenous c-met ligand for use according to claim 7, wherein the effects and/or biological activity of endocrine-produced HGF in the individual are enhanced, and optionally the effects and/or biological activity of autocrinally produced HGF are inhibited.

9. Endogenous c-met ligand for use in a method for affecting angiogenesis, cell division, migration and/or morphogenesis in an individual, said method comprising administering to the individual an effective amount of endogenous c-met ligand, wherein the endogenous c-met ligand is a native or recombinant human anti c-met immunoglobulin.

10. A method for determining the amount of endogenous human anti c-met immunoglobulin in a serum sample from an individual, comprising analyzing said serum sample using Surface Plasmon Resonance (SPR).

11. A method for determining if an individual is a candidate for treatment with endogenous human c-met ligand, comprising determining the amount of endogenous human c-met ligand in a serum sample from the individual and comparing the determined amount with an amount of endogenous human c-met ligand representative of healthy individuals, wherein a determined amount lower than the representative amount indicates the individual as a candidate for treatment with endogenous human c-met ligand, wherein said candidate optionally is a patient with an autoimmune disease.

12. Endogenous human anti c-met immunoglobulin isolated from a serum sample from a healthy individual or an immunoglobulin preparation.

## Patentansprüche

1. Endogener c-Met-Ligand oder ein Fragment davon zur Verwendung in einem Verfahren zur Inhibition von Karzinogenese und/oder Metastasierung in einem Individuum, umfassend die Verabreichung einer effektiven Menge des endogenen c-Met-Liganden oder des Fragments davon dem Individuum, wobei der endogene c-Met-Ligand ein natives oder rekombinantes menschliches anti-c-Met Immunglobulin ist.

2. Endogener c-Met-Ligand oder ein Fragment davon gemäß Anspruch 1, **dadurch gekennzeichnet, dass** der endogene c-Met-Ligand ein natives menschliches anti-c-Met Immunglobulin ist, erhalten aus Serum eines gesunden Individuums oder einer Immunglobulin-Zubereitung.

3. Endogener c-Met-Ligand oder ein Fragment davon gemäß Anspruch 1, wobei der endogene c-Met-Ligand oder das Fragment davon autokrine HGF-produzierende Zellen in dem Individuum inhibiert, und optional der endogene c-Met-Ligand endokrine HGF-produzierende Zellen in dem Individuum nicht inhibiert.

4. Endogener c-Met-Ligand oder ein Fragment davon gemäß Anspruch 3, wobei wobei die Effekte der endokrinen HGFproduzierenden Zellen in dem Individuum verstärkt werden.

5. Endogener c-Met-Ligand oder ein Fragment davon gemäß Anspruch 1, zur Verwendung in einem Verfahren zur Inhibition von Karzinogenese und/oder Metastasierung in einem Individuum, wobei der endogene c-Met-Ligand oder das Fragment davon vor, während und/oder nach Operation und/oder nachdem der Tumor entfernt ist verabreicht wird.

6. Endogener c-Met-Ligand oder ein Fragment davon gemäß Anspruch 5, zur Verwendung in einem Verfahren zur Inhibition von Karzinogenese und/oder Metastasierung in einem Individuum, wobei die Krebstherapie Operation umfasst.

7. Endogener c-Met-Ligand zur Verwendung in einem Verfahren zur Inhibition der Interaktion von autokrin produziertem HGF mit dem c-Met Rezeptor in einem Individuum, umfassend die Verabreichung einer effektiven Menge des endogenen c-Met-Liganden dem Individuum, wobei der endogene c-Met-Ligand ein natives oder rekombinantes menschliches anti-c-Met Immunglobulin ist.

8. Endogener c-Met-Ligand zur Verwendung gemäß Anspruch 7, wobei die Effekte und/oder die biologische Aktivität des endokrin produzierten HGF in dem Individuum verstärkt werden, und optional die Effekte und/oder die biologische Aktivität des autokrin produzierten HGF inhibiert werden.

9. Endogener c-Met-Ligand zur Verwendung in einem Verfahren zur Beeinflussung von Angiogenese, Teilung von Zellen, Migration und/oder Morphogenese in einem Individuum, wobei das Verfahren die Verabreichung einer effektiven Menge des endogenen c-Met-Liganden dem Individuum umfasst, wobei der endogene c-Met-Ligand ein natives oder rekombinantes menschliches anti-c-Met Immunglobulin ist.

10. Verfahren zur Bestimmung der Menge von endogenem menschlichem anti-c-Met-Immunglobulin in einer Serumprobe aus einem Individuum, umfassend die Analyse der Serumprobe mittels Oberflächenplasmonenresonanz (Surface Plasmon Resonance, SPR).

11. Verfahren zur Bestimmung, ob ein Individuum ein Kandidat für die Behandlung mit endogenem menschlichem c-Met-Liganden ist, umfassend die Bestimmung der Menge von endogenem menschlichem c-Met-Liganden in einer Serumprobe aus dem Individuum und das Vergleichen der bestimmten Menge mit einer für gesunde Individuen repräsentativen Menge von endogenem menschlichem c-Met-Liganden, wobei eine bestimmte Menge, die niedriger als die repräsentative Menge ist, anzeigt, dass das Individuum ein Kandidat für die Behandlung mit endogenem menschlichem c-Met-Liganden ist, wobei der Kandidat optional ein Patient mit einer Autoimmunerkrankung ist.

12. Endogenes menschliches anti-c-Met Immunglobulin, isoliert aus einer Serumprobe aus einem gesunden Individuum oder einer Immunglobulin-Zubereitung.

## Revendications

1. Ligand c-met endogène ou fragment de celui-ci pour une utilisation dans un procédé d'inhibition de la carcinogenèse et/ou de la métastase chez un individu, comprenant l'administration à l'individu d'une quantité efficace dudit ligand c-met endogène ou du fragment de celui-ci, dans lequel le ligand c-met endogène est une immunoglobuline anti-c-met humaine native ou recombinante.

2. Ligand c-met endogène ou fragment de celui-ci selon la revendication 1, **caractérisé en ce que** le ligand c-met endogène est une immunoglobuline anti-c-met humaine native obtenue à partir du sérum d'un individu sain ou d'une préparation d'immunoglobulines.

3. Ligand c-met endogène ou fragment de celui-ci selon la revendication 1, dans lequel ledit ligand c-met endogène ou fragment de celui-ci inhibe les cellules productrices de HGF autocrines chez l'individu, et éventuellement le ligand c-met endogène n'inhibe pas les cellules productrices de HGF endocrines chez l'individu.

4. Ligand c-met endogène ou fragment de celui-ci selon la revendication 3, dans lequel les effets des cellules productrices de HGF endocrines chez l'individu sont renforcés.

5. Ligand c-met endogène ou fragment de celui-ci selon la revendication 1, pour utilisation dans un procédé d'inhibition de la carcinogenèse et/ou de la métastase chez un individu, dans lequel ledit ligand c-met endogène ou fragment de celui-ci est administré avant, pendant et/ou après une opération chirurgicale et/ou après que la tumeur est enlevée.

6. Ligand c-met endogène ou fragment de celui-ci selon la revendication 5, pour utilisation dans un procédé d'inhibition de la carcinogenèse et/ou de la métastase chez un individu, dans lequel ladite thérapie du cancer comprend une opération chirurgicale.

7. Ligand c-met endogène pour utilisation dans un procédé d'inhibition de l'interaction du HGF produit de manière autocrine avec le récepteur c-met chez un individu, comprenant l'administration à l'individu d'une quantité efficace dudit ligand c-met endogène, dans lequel le ligand c-met endogène est une immunoglobuline anti-c-met humaine native ou recombinante.

8. Ligand c-met endogène pour utilisation selon la revendication 7, dans lequel les effets et/ou l'activité biologique du HGF produit de manière endocrine chez l'individu sont renforcés, et éventuellement les effets et/ou l'activité biologique du HGF produit de manière autocrine sont inhibés.

9. Ligand c-met endogène pour utilisation dans un procédé permettant d'affecter l'angiogenèse, la division cellulaire, la migration et/ou la morphogenèse chez un individu, ledit procédé comprenant l'administration à l'individu d'une quantité efficace de ligand c-met endogène, dans lequel le ligand c-met endogène est une immunoglobuline anti-c-met humaine native ou recombinante.

10. Procédé de détermination de la quantité d'immunoglobuline anti-c-met humaine endogène dans un échantillon de sérum provenant d'un individu, comprenant l'analyse dudit échantillon de sérum par résonance plasmonique de surface (RPS).

11. Procédé permettant de déterminer si un individu est un candidat pour un traitement avec un ligand c-met humain endogène, comprenant la détermination de la quantité de ligand c-met humain endogène dans un échantillon de sérum provenant de l'individu et la comparaison de la quantité déterminée avec une quantité de ligand c-met humain endogène représentative d'individus sains, dans lequel une quantité déterminée inférieure à la quantité représentative indique que l'individu est un candidat pour un traitement avec le ligand c-met humain endogène, dans lequel ledit candidat est éventuellement un patient atteint d'une maladie auto-immune.

12. Immunoglobuline anti-c-met humaine endogène isolée à partir d'un échantillon de sérum issu d'un individu sain ou d'une préparation d'immunoglobuline.
